# EUROPÄISCHE PATENTANMELDUNG

(11) **EP 2 138 842 A1**
(43) Veröffentlichungstag der Anmeldung: **30.12.2009**
(21) Anmeldenummer: 08158943.4
(22) Anmeldetag: 25.06.2008
(51) Int. Cl.: G01N 33/487

(54) **Analysehandgerät zur Untersuchung einer Körperflüssigkeit und Steuerverfahren dafür**

(71) Anmelder: F. Hoffmann-La Roche AG, 4070 Basel (CH); Roche Diagnostics GmbH, 68305 Mannheim (DE)
(72) Erfinder: Miltner, Karl, D-67227 Frankenthal (DE); Liedtke, Sebastian, D-69117 Heidelberg (DE); Baeter, Thorsten, D-67136 Fussgönheim (DE); Schmid, Wilfried, D-68165 Mannheim (DE); Frisch, Gerhard, D-68535 Edingen-Neckarhausen (DE); Heck, Wolfgang, D-68526 Ladenburg (DE)
(74) Vertreter: Pfiz, Thomas

(57) **Zusammenfassung**

Die Erfindung betrifft ein Analysehandgerät (10) zur Untersuchung einer Körperflüssigkeit, bei dem mittels einer Steuereinrichtung (16) in aufeinanderfolgenden Messzyklen jeweils mindestens ein Testmittel (44) für den Einmalgebrauch automatisch bereitgestellt wird, wobei jeder Messzyklus in einem Messbetrieb der Steuereinrichtung (16) durch eine Startaktuation ausgelöst wird. Erfindungsgemäß wird vorgeschlagen, dass die Steuereinrichtung (16) durch einen definierten Steuereingriff in einen Wartungsbetrieb versetzt wird, in welchem die automatische Bereitstellung eines Testmittels (44) verhindert wird. Die Erfindung betrifft weiterhin ein entsprechendes Steuerungsverfahren für das Gerät (10).

## Beschreibung

Die Erfindung betrifft ein Verfahren zur Steuerung eines Analysehandgeräts zur Untersuchung einer Körperflüssigkeit, insbesondere für Blutzuckertests, bei dem mittels einer Steuereinrichtung in aufeinanderfolgenden Messzyklen jeweils mindestens ein Testmittel für den Einmalgebrauch automatisch bereitgestellt wird, wobei jeder Messzyklus in einem Messbetrieb der Steuereinrichtung durch eine Startaktuation ausgelöst wird. Die Erfindung betrifft weiter ein Analysehandgerät zur Untersuchung einer Körperflüssigkeit.

Ein solches Verfahren geht aus der EP-A 1 770 395 hervor, bei dem einzelne Teststreifen aus einem wechselbaren Trommelmagazin für Analysezwecke bereitgestellt werden. Dort erfolgt durch Zuordnen eines Zählerstandes zu einer Magazinkennung eine geräteseitige Verbrauchserfassung unter Rückschreibeverzicht auf das Magazin, so dass auch der wahlweise Einsatz von teilverbrauchten Magazinen vereinfacht wird.

Zur weiteren Systemintegration wurde beispielsweise in der EP-A 1 739 432 bereits vorgeschlagen, anstelle einzelner Streifen ein aufgewickeltes Testband mit beabstandeten analytischen Testfeldern als Testmittel in einem Handgerät einzusetzen. Zur Wegerfassung beim Bandtransport sind dort Markierungsbereiche vorgesehen, die eine exakte Positionierung der Testfelder erlauben. Bei solchen Konzepten stellt die Minimierung der Baugröße und der erforderlichen Bedienschritte eine besondere Herausforderung dar.

Ausgehend hiervon liegt der Erfindung die Aufgabe zugrunde, die im Stand der Technik bekannten Verfahren und Erzeugnisse weiter zu entwickeln und mit einfachen Mitteln eine erhöhte Benutzerfreundlichkeit und Verfahrenssicherheit auch hinsichtlich einer optimierten Nutzung der Testmittel zu erreichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von der Überlegung aus, dass die Bereitstellung eines analytischen Testmittels in einem Handgerät in der Regel nicht rückgängig gemacht werden kann, ohne die Testqualität zu mindern. Diese Problematik ist vergleichbar mit herkömmlichen Filmkameras, die bei eingelegtem Film nicht einfach geöffnet werden sollten. Dementsprechend wird in verfahrensmäßiger Hinsicht vorgeschlagen, dass die Steuereinrichtung durch einen definierten Steuereingriff in einen Wartungsbetrieb versetzt wird, in welchem die automatische Bereitstellung eines Testmittels verhindert wird. Auf diese Weise wird ein ungewollter Testverlust vermieden, ohne dass die Handhabung im normalen Messablauf beeinträchtigt würde.

Vorteilhafterweise wird der Start eines Messzyklus im Wartungsbetrieb auch bei einer Startaktuation blockiert. Somit können Handhabungsschritte vorgenommen werden, die im Messbetrieb zu einem Start der Messung führen würden.

Zweckmäßig ist der Wartungsbetrieb dazu eingerichtet, eine Gerätereinigung oder einen Testmittelaustausch ohne Testmittelverlust zu ermöglichen.

Um den Bauaufwand zu reduzieren, ist es vorteilhaft, wenn der Wartungsbetrieb über ein Softwaremenü der elektronischen Steuereinrichtung durch einen Benutzer ausgewählt wird. Dies lässt sich besonders einfach dadurch realisieren, dass der Wartungsbetrieb über einen manuell betätigbaren Schalter, insbesondere einen Softkey eingeleitet wird.

Eine weitere vorteilhafte Ausgestaltung sieht vor, dass eine vorbestimmte Betätigung eines Gerätebauteils durch die Steuereinrichtung als Startaktuation erkannt wird. Auf diese Weise kann die Anzahl der erforderlichen Bedienschritte reduziert werden und eine schnelle Verfügbarkeit erreicht werden. In diesem Zusammenhang ist es auch günstig, wenn die Startaktuation und/oder ein Austausch der Testmittel durch einen Sensor überwacht wird, und wenn im Wartungsbetrieb das Sensorsignal ignoriert wird.

Vorteilhafterweise werden die Testmittel in einem Gehäuse des Analysehandgeräts gegen Umwelteinflüsse, insbesondere Feuchtigkeitszutritt geschützt, um eine hohe Zuverlässigkeit zu gewährleisten.

Eine weitere Verbesserung der Bedienfreundlichkeit lässt sich dadurch erreichen, dass die Messzyklen durch das Öffnen einer Schutzabdeckung des Analysehandgeräts ausgelöst werden, wobei ein Testmittel für einen Benutzerzugriff bereitgestellt wird.

Vor allem für den Einsatz von magazinierten Testmitteln ist es vorteilhaft, wenn die Testmittel im Messbetrieb durch eine Transporteinrichtung an mindestens eine vorgegebene Position transportiert werden, und wenn der automatische Testmitteltransport im Wartungsbetrieb unterbrochen wird. Gemäß einer besonderen Ausgestaltung werden während eines Messzyklus die jeweiligen Testmittel von einem Testmittelvorrat zu einer Applikationsstelle und gegebenenfalls zu einem Testmittelabfall transportiert.

Eine besonders vorteilhafte Anwendung besteht darin, dass ein mit einer Vielzahl von analytischen Testfeldern und/oder Stechelementen als Testmittel bestücktes Testband eingesetzt wird, und dass das Testband zur Bereitstellung der Testmittel abschnittsweise vorgespult wird. Eine weitere Verbesserung wird dadurch erreicht, dass die Testmittel vorzugsweise in Form eines Magazins oder einer Kassette in ein Gerätefach eingewechselt werden, und dass im Messbetrieb beim Öffnen des Gerätefachs ein durch eine Startaktuation aktiviertes Testmittel verworfen wird.

Gegenstand der Erfindung ist auch eine besondere Vorrichtung zur Durchführung des Verfahrens umfassend eine Steuereinrichtung zur automatischen Bereitstellung von für den Einmalgebrauch ausgebildeten Testmitteln in aufeinanderfolgenden Messzyklen, wobei jeder Messzyklus in einem Messbetrieb der Steuereinrichtung durch eine Startaktuation auslösbar ist, und wobei die Steuereinrichtung durch einen definierten Steuereingriff in einen Wartungsbetrieb versetzbar ist, in welchem die automatische Bereitstellung eines Testmittels verhindert wird.

Im Folgenden wird die Erfindung anhand des in der Zeichnung schematisch dargestellten Ausführungsbeispiels näher erläutert. Es zeigen:
- Fig. 1: ein tragbares Blutzuckermessgerät zum Einsatz einer Testbandkassette in perspektivischer Ansicht;
- Fig. 2: das Messgerät nach Fig. 1 in einem Längsschnitt;
- Fig. 3: ein Flussdiagramm zur Veranschaulichung des Mess- und Wartungsbetriebs des Blutzuckermessgeräts.

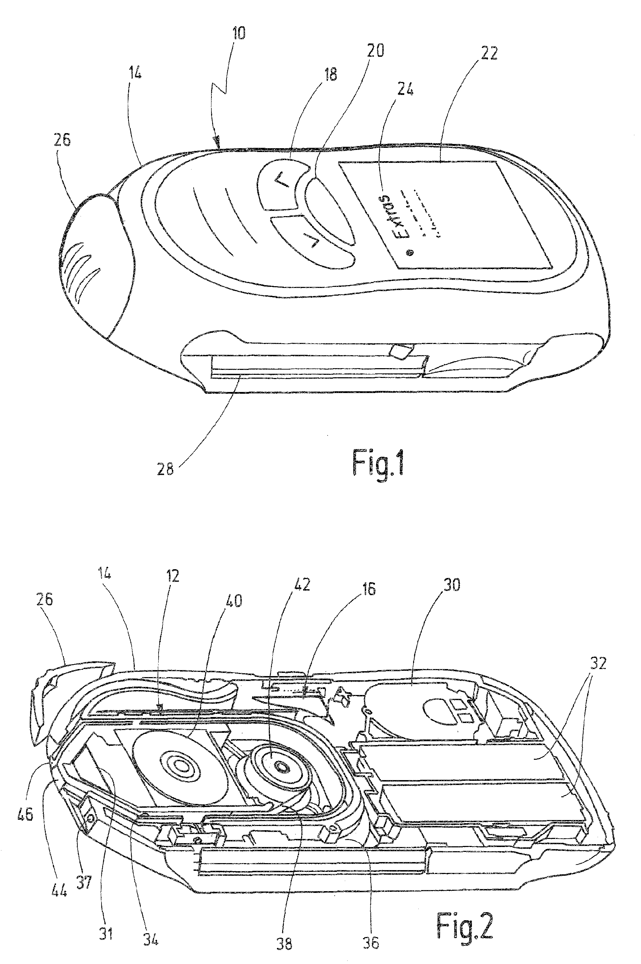

Das in Fig. 1 und 2 dargestellte tragbare Blutzuckermessgerät 10 erlaubt einem Benutzer die Selbstbestimmung des Blutzuckerspiegels vor Ort in einem weitgehend automatischen Messablauf. Dabei sind eine Vielzahl von analytischen Testmitteln in Form einer Bandkassette 12 in das kompakte Gerätegehäuse 14 einsetzbar, um ohne aufwändige Handhabung entsprechend viele Tests durchführen zu können. Für die Gerätewartung kann ein ungewollter Testmittelverlust durch eine vorteilhafte Ablaufsteuerung mittels einer elektronischen Steuereinrichtung 16 verhindert werden.

Wie aus Fig. 1 ersichtlich, sind an der Oberseite des Gehäuses 14 verschiedene Steuertasten 18, 20 zur manuellen Betätigung angeordnet. Ein Bildschirm 22 ermöglicht die Anzeige von Bedienmenüs 24 und von Messergebnissen. Die in dem Gehäuse 14 geschützte Bandkassette 12 ist durch Öffnen einer Schutzabdeckung 26 an einer Applikationsstelle für den Auftrag einer Blutprobe zugänglich. Eine zur Gewinnung der Blutprobe vorgesehene Stechhilfe (nicht gezeigt) lässt sich an einer Halterung 28 seitlich an dem Gehäuse 14 anflanschen.

Fig. 2 zeigt im Schnitt das Geräteinnere mit eingesetzter Bandkassette 12, Steuereinrichtung (Platine 16), Bandantriebseinheit 30, Messeinheit 31 und Energieversorgung (Batterien 32). Die in dem Kassettenfach 34 gelagerte Kassette 12 ist als Verbrauchsartikel über einen bodenseitigen Gehäusedeckel 36 auswechselbar. Die Stellung der verschiednen Gerätezugänge wird sensorisch erfasst, wie dies für die Schutzabdeckung 26 durch den Schalter 37 beispielhaft dargestellt ist.

Die Kassette 12 umfasst ein Testband 38, eine nach außen abgedichtete Vorratsspule 40 zum Abwickeln von ungebrauchtem Testband und eine Aufwickelspule 42 zum Aufwickeln von gebrauchtem Testband. Auf dem Testband 38 sind mit einer Testchemie beschichtete Testfelder 44 als Testmittel zum Nachweis des Analyten (Glukose) im Abstand voneinander aufgebracht. Alternativ oder ergänzend könnten auch nicht gezeigte Stechelemente für einen Hauteinstich auf einem Trägerband magaziniert werden. Das Testband 38 lässt sich mittels der Antriebseinheit 30 vorspulen, so dass die Testeinheiten 20 an einer durch Wegschwenken der Schutzabdeckung 26 freigegebenen Applikationsstelle 46 für die Blutprobenauftragung sukzessive bereitstellbar sind. Dabei kann durch eine rückseitige reflektometrische Vermessung des mit Blut beaufschlagten Testfeldes 44 anhand eines Farbumschlags ein Glukosemesswert gewonnen werden.

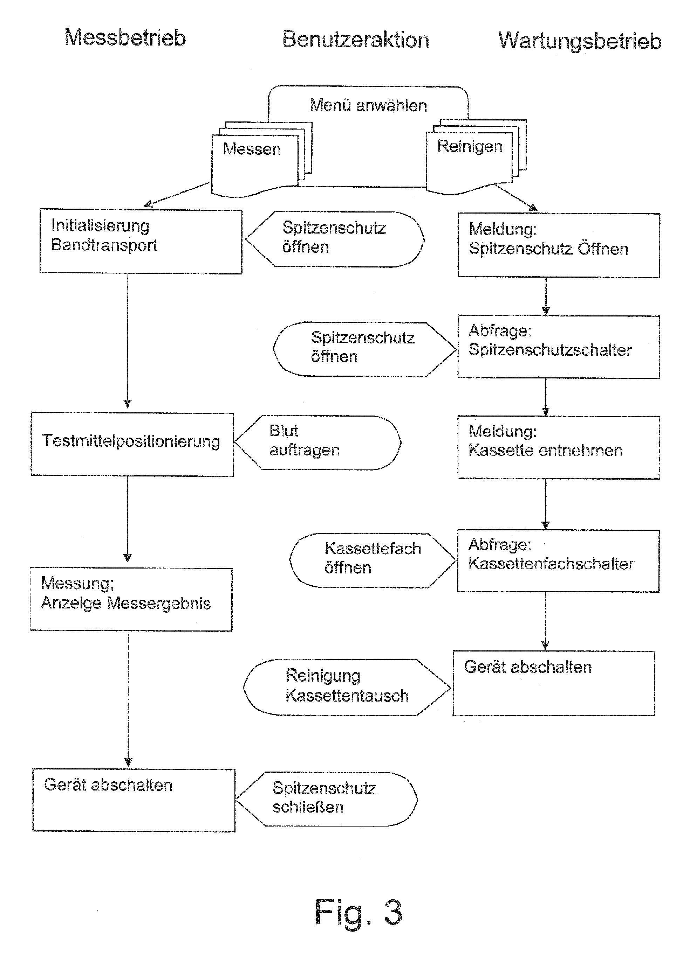

Bei der in Fig. 3 veranschaulichten Ablaufsteuerung wird durch einfache Maßnahmen ein Alternativbetrieb zwischen Messen und Wartung unter Minimierung der erforderlichen Bedienschritte bzw. Benutzeraktion ermöglicht, wobei ein Testmittelverlust weitgehend vermieden wird.

Wie im linken Teil des Diagramms nach Fig. 3 dargestellt, kann ein Messzyklus durch Öffnen des Spitzenschutzes (Schutzabdeckung 26) vom Benutzer gestartet werden, ohne dass zusätzliche Einschaltknöpfe zu bedienen wären. Damit ist eine sehr rasche Verfügbarkeit des Systems gewährleistet. Als alternative Startaktuation kann aber auch die Menüauswahl "Messen" über die Bedientasten 18, 20 gewählt werden.

Bis zum Start des Messzyklus bleiben die unbenutzen Testfelder 44 auf der nach außen abgedichteten Vorratsspule 40 gegen Umgebungseinflüsse wie Licht, Feuchte, Schmutz abgeschirmt. Die Testfeldpositionierung an der Applikationsstelle 46 wird dann durch Bandtransport gesteuert, wobei während einer Initialisierungsphase auf dem Testband 38 aufgebrachte Steuermarken erfasst werden, um ein definiertes Vorspulen zu ermöglichen. Ein eventuelles Rückspulen ist hier wegen des apparativen Aufwandes nicht vorgesehen und könnte auch zu undefinierten Testmittelzuständen führen. Als nächste Benutzeraktion erfolgt anschließend der Blutauftrag auf das über der Messspitze positionierte Testfeld 44. Sodann kann ein Messergebnis mittels Messeinheit 31 gewonnen und auf der Anzeige 22 angezeigt werden. Der Messzyklus wird auf einfache Weise durch Schließen des Spitzenschutzes 26 durch den Benutzer beendet, wobei das benutzte Testfeld 44 durch weiteren Bandtransport auf der Aufwickelspule 42 entsorgt und das Gerät abgeschaltet wird. Ein solcher Messzyklus lässt sich bis zum Verbrauch aller bevorrateten Testfelder 44 wiederholen, bevor ein Kassettenwechsel notwendig ist.

Sofern im Messbetrieb während eines Messzyklus ein nicht vorgesehener, aber überwachter Gerätezugriff erfolgt, insbesondere das Kassettenfach 34 geöffnet wird, verwirft die Steuereinrichtung aus Sicherheitsgründen den laufenden Test, um eine eventuelle Messwertverfälschung auszuschließen. Hier ist zu bedenken, dass die Gerätebedienung auch für Laien ausgelegt sein muss, und dass ein falsches Messergebnis zu schwerwiegenden Fehldiagnosen führen kann.

Zu unterscheiden davon ist ein notwendiger Zugriff auf das Geräteinnere, um eine Reinigung vorzunehmen oder andere Servicefunktionen bzw. Demonstrationszwecke zu erfüllen. In einem solchen Fall soll ein nachteiliger Verlust eines Testmittels bzw. Testfelds 44 vermieden werden. Hierfür lässt sich die Steuereinrichtung durch einen definierten Steuereingriff in einen Wartungsbetrieb versetzten, wie im rechten Diagrammteil der Fig. 3 gezeigt.

Der Steuereingriff wird durch den Menüpunkt "Reinigen" eines Softwaremenüs realisiert, das der Benutzer über die Bedientaste 20 einfach aufrufen kann. Diese Taste ist als "Softkey" ausgelegt und kann somit abhängig von einer Bildschirmanzeige unterschiedliche Funktionen ausüben.

Sobald der Wartungsbetrieb eingeleitet ist, werden die Sensorsignale beim Öffnen des Spitzesschutzes 26 oder des Kassettenfachs 34 ignoriert bzw. nicht mehr als Startaktuation interpretiert, so dass kein automatischer Bandtransport mehr erfolgt. Der Start eines Messzyklus wird somit blockiert, und ein ungewollter Testmittelverlust wird verhindert.

Nun kann der Benutzer über den Bildschirm 22 zur Vornahme bestimmter Handlungen aufgefordert werden. Nach Öffnen des Spitzenschutzes 26 und des Kassettenfachdeckels 36 wird das Gerät abgeschaltet. Der Benutzer kann dann die Kassette 12 entnehmen, den Geräteinnenraum und insbesondere die Messoptik 31 reinigen, die Kassette wieder einlegen und Kassettenfach sowie Spitzenschutz wieder schließen. Das Gerät 10 befindet sich dann wieder in Bereitschaft für den Messbetrieb.

## Patentansprüche

1. Verfahren zur Steuerung eines Analysehandgeräts (10) zur Untersuchung einer Körperflüssigkeit, insbesondere für Blutzuckertests, bei dem mittels einer Steuereinrichtung (16) in aufeinanderfolgenden Messzyklen jeweils mindestens ein Testmittel (44) für den Einmalgebrauch automatisch bereitgestellt wird, wobei jeder Messzyklus in einem Messbetrieb der Steuereinrichtung (16) durch eine Startaktuation ausgelöst wird, **dadurch gekennzeichnet, dass** die Steuereinrichtung (16) durch einen definierten Steuereingriff in einen Wartungsbetrieb versetzt wird, in welchem die automatische Bereitstellung eines Testmittels (44) verhindert wird.

2. Verfahren nach Anspruch 1, **dadurch gekennzeichnet, dass** der Start eines Messzyklus im Wartungsbetrieb auch bei einer Startaktuation blockiert wird.

3. Verfahren nach Anspruch 1 oder 2, **dadurch gekennzeichnet, dass** im Wartungsbetrieb eine Gerätereinigung oder ein Testmittelaustausch ohne Testmittelverlust ermöglicht wird.

4. Verfahren nach einem der Ansprüche 1 bis 3, **dadurch gekennzeichnet, dass** der Wartungsbetrieb über ein Softwaremenü (24) der elektronischen Steuereinrichtung (16) durch einen Benutzer ausgewählt wird.

5. Verfahren nach einem der Ansprüche 1 bis 4, **dadurch gekennzeichnet, dass** der Wartungsbetrieb über einen manuell betätigbaren Schalter (20), insbesondere einen Softkey eingeleitet wird.

6. Verfahren nach einem der Ansprüche 1 bis 5, **dadurch gekennzeichnet, dass** eine vorbestimmte Betätigung eines Gerätebauteils durch die Steuereinrichtung (16) als Startaktuation erkannt wird.

7. Verfahren nach einem der Ansprüche 1 bis 6, **dadurch gekennzeichnet, dass** die Startaktuation und/oder ein Austausch der Testmittel (44) durch einen Sensor (37) überwacht wird, und dass im Wartungsbetrieb das Sensorsignal ignoriert wird.

8. Verfahren nach einem der Ansprüche 1 bis 7, **dadurch gekennzeichnet, dass** die Testmittel (44) in einem Gehäuse (14) des Analysehandgeräts gegen Umwelteinflüsse, insbesondere Feuchtigkeitszutritt geschützt werden.

9. Verfahren nach einem der Ansprüche 1 bis 8, **dadurch gekennzeichnet, dass** die Messzyklen durch das Öffnen einer Schutzabdeckung (26) des Analysehandgeräts ausgelöst werden, wobei ein Testmittel (44) für einen Benutzerzugriff bereitgestellt wird.

10. Verfahren nach einem der Ansprüche 1 bis 9, **dadurch gekennzeichnet, dass** die Testmittel (44) im Messbetrieb durch eine Transporteinrichtung (30) an mindestens eine vorgegebene Position transportiert werden, und dass der automatische Testmitteltransport im Wartungsbetrieb unterbrochen wird.

11. Verfahren nach einem der Ansprüche 1 bis 10, **dadurch gekennzeichnet, dass** während eines Messzyklus die jeweiligen Testmittel (44) von einem Testmittelvorrat (40) zu einer Applikationsstelle (46) und gegebenenfalls zu einem Testmittelabfall (42) transportiert werden.

12. Verfahren nach einem der Ansprüche 1 bis 11, **dadurch gekennzeichnet, dass** ein mit einer Vielzahl von analytischen Testfeldern und/oder Stechelementen als Testmittel (44) bestücktes Testband (38) eingesetzt wird, und dass das Testband (38) zur Bereitstellung der Testmittel (44) abschnittsweise vorgespult wird.

13. Verfahren nach einem der Ansprüche 1 bis 12, **dadurch gekennzeichnet, dass** die Testmittel (44) vorzugsweise in Form eines Magazins oder einer Kassette (12) in ein Gerätefach (34) eingewechselt werden, und dass im Messbetrieb beim Öffnen des Gerätefachs (34) ein durch eine Startaktuation aktiviertes Testmittel (44) verworfen wird.

14. Analysehandgerät zur Untersuchung einer Körperflüssigkeit, insbesondere für Blutzuckertests, mit einer Steuereinrichtung (16) zur automatischen Bereitstellung von für den Einmalgebrauch ausgebildeten Testmitteln (44) in aufeinanderfolgenden Messzyklen, wobei jeder Messzyklus in einem Messbetrieb der Steuereinrichtung (16) durch eine Startaktuation auslösbar ist, **dadurch gekennzeichnet, dass** die Steuereinrichtung (16) durch einen definierten Steuereingriff in einen Wartungsbetrieb versetzbar ist, in welchem die automatische Bereitstellung eines Testmittels (44) verhindert wird.
